# EUROPEAN PATENT SPECIFICATION

(11) **EP 2 579 949 B1**
(45) Date of publication and mention of the grant of the patent: **14.08.2019**
(21) Application number: 11725587.7
(22) Date of filing: 03.06.2011
(51) Int. Cl.: A61Q 5/12

(54) **PERSONAL CARE COMPOSITION COMPRISING A SURFACTANT-RICH GEL NETWORK**
KÖRPERPFLEGEZUSAMMENSETZUNG UMFASSEND EIN TENSIDREICHES GELNETZWERK
COMPOSITION D'HYGIÈNE CORPORELLE COMPRENANT UN RÉSEAU DE GEL RICHE EN TENSIOACTIF

(30) Priority: 10.06.2010 US 353465 P
(43) Date of publication of application: 17.04.2013
(73) Proprietor: The Procter & Gamble Company, Cincinnati, OH 45202 (US)
(72) Inventor: WISE, Geoffrey, Marc, Reading Ohio 45215 (US); WOOD, Randall, Lee, Springboro Ohio 45066 (US); CHU, Long, Van, Mason Ohio 45040 (US)
(74) Representative: Sauvaître, Thibault Bruno
(86) International application number: PCT/US2011/039026
(87) International publication number: WO 2011/156217

(56) References cited:
- EP-A2- 0 627 216
- US-A- 4 772 427
- US-A1- 2003 223 952
- US-A1- 2006 269 501
- US-A1- 2006 269 502
- US-A1- 2010 029 528
- "Stearic Acid" In: "International Cosmetic Ingredient Dictionary and Handbook", 2008, The Cosmetic, Toiletry, and Fragrance Association, XP002705571, vol. 2, pages 2658-2659, paragraph "Functions"

## Description

### TECHNICAL FIELD OF THE INVENTION

Embodiments of the present disclosure are generally related to personal care compositions and processes of making same, and are specifically related to personal care compositions comprising surfactant-rich gel networks.

### BACKGROUND OF THE INVENTION

For personal care formulas, such as cosmetic creams and hair conditioners, it is known to include gel network premixes that yield conditioning benefits to the personal care formula. U.S.

Patent Application US 2006/0269502 A1 refers to shampoo compositions comprise (a) from 5% to 50% of one or more detersive surfactants; (b) a dispersed gel network phase comprising: (i) at least 0.05% of one or more fatty amphiphiles; (ii) at least 0.01% of one or more secondary surfactants; and (iii) water; (c) at least 0.05% of a galactomannan polymer derivative with a net positive charge and having a mannose to galactose ratio of greater than 2:1 on a monomer to monomer basis, and (d) at least 20% of an aqueous carrier; all by weight of the shampoo composition.

Wells et al. US 7,303,744 (hereinafter the '744 patent) discloses the manufacture of fatty-alcohol/surfactant gel networks premixes to be added to personal care base formulations for conditioning benefits. The formation of a gel network involves heating a dispersion of the fatty amphiphile with a surfactant to a temperature above the melting point of the fatty amphiphile. When the mixture is cooled, it is converted to a solid crystalline gel network.

The '744 patent teaches a gel network premix having a weight ratio of fatty amphiphile to surfactant (FA:S) ratio of 1:1 or greater. If increasing levels of surfactants are used to form the gel network premixes, the hot gel network premix increases in viscosity, and becomes difficult to handle and process. The increasing rheology, and handling difficulty is exemplified by Table 1, which lists the approximate agitation speed (in revolutions per minute) required for adequate mixing of various compositions of fatty amphiphiles and surfactants. In this example, the fatty amphiphile was a 1.8:1 mixture (by weight) of stearyl alcohol and cetyl alcohol, the surfactant was sodium laureth-3 sulfate, and the mixing experiments were conducted in a 75°C 30-liter vessel.

**TABLE 1: RPM Required to Mix Gel Network Premixes**

| Active fatty amphiphile | Active surfactant | FA:S Ratio | Required RPM to mix |
|---|---|---|---|
| 11.7 % | 5.0 % | 2.34 | 120 |
| 11.7 % | 8.0 % | 1.46 | 230 |
| 14.0 % | 4.0 % | 3.50 | 115 |
| 14.0 % | 6.0 % | 2.33 | 150 |
| 14.0 % | 8.0 % | 1.75 | 290 |

Table 1 demonstrates that increasing the surfactant level at a given fatty amphiphile level increases the difficulty of mixing the different compositions to form hot gel network premixes, such that concentrated premixes at FA:S ratios below 1:1 have been considered impractical due to the high level of liquid crystalline phase in the hot premix.

On the other hand, we have determined that the trend of rheology versus FA:S ratio is often reversed after the cooling step. Cooled gel network premix having a weight ratio of fatty amphiphile to surfactant (FA: S) ratio of 1:1 or greater are difficult to homogeneously disperse into personal care component to form the personal care composition due to the high viscosity and rheology of the cooled gel network premix. The difficult rheology and viscosity of the cooled premix creates unpredictability when dispersing the gel network premix with personal care components, as well as consuming additional time to ensure an adequate dispersion.

Accordingly, there remains a need for improved gel network premixes that may be more easily dispersed in a personal care formulation with greater formulation flexibility.

### SUMMARY OF THE INVENTION

Surprisingly, the applicants have discovered that surfactant rich gel network premixes having a fatty amphiphile to surfactant ratio (FA:S) by weight of less than 1:1 can be effectively formed, and homogeneously dispersed with personal care components to form a personal care composition. Fatty amphiphile rich gel network premixes having a weight ratio of fatty amphiphile to surfactant (FA:S) of less than 1:1 allows greater formulation flexibility, ease of storage and handling, and facile dispersion of the gel-network premix into personal care components. In addition, gel network premixes having a ratio of fatty amphiphile to surfactant (FA:S) below 1:1 experience smaller melting-point and composition changes upon blending into the personal care components to produce the personal care composition, thus reducing the equilibration time required to achieve the final viscosity and gel network structure in the personal care composition.

A process for preparing a personal care composition is provided, said process comprising:
combining a fatty amphiphile above its melt temperature with a surfactant in a fatty amphiphile to surfactant weight ratio from 0.75:1 to 0.9:1 to form a gel network premix, wherein the fatty amphiphile and surfactant are combined at a temperature above the melt temperature of the fatty amphiphile or the melt temperature of a blend of fatty amphiphiles,
   wherein the fatty amphiphile comprises from 10 to 40 carbon atoms, and wherein the fatty amphiphile is straight or branched chain alcohols and are saturated or unsaturated,
   wherein the surfactant is selected from the group consisting of alkyl sulfate, alkyl ether sulfate, and combinations thereof;
contacting the gel network premix with a shearing device;
cooling the gel network premix after contact with the shearing device to a temperature below the melt temperature of the fatty amphiphile; and
adding the cooled gel network premix to one or more personal care components to form the personal care composition, wherein the final personal care composition comprises from 10 wt% to 50 wt% of the gel network premix.

### BRIEF DESCRIPTION OF THE DRAWINGS

The following detailed description of specific embodiments of the present disclosure can be best understood when read in conjunction with the following drawings, and in which:
Fig. 1 is a generalized phase diagram illustrating the equilibrium of a gel network premix in a personal care composition in accordance with one embodiment;
Fig. 2A and 2B are polarized light microscopy views depicting two different compositions of both a high and low fatty amphiphile to surfactant ratio gel network premix in accordance with one embodiment; and
Fig. 3 is a graphical illustration providing a comparison of the kinetics for dispersing both a high and low fatty amphiphile to surfactant ratio gel network premix into personal care components to form a personal care composition in accordance with another embodiment.

### DETAILED DESCRIPTION OF THE INVENTION

All percentages, parts, and ratios are based upon the total weight of the compositions of the present invention, unless otherwise specified. All such weights as they pertain to listed ingredients are based on the active level and therefore; do not include solvents or by-products that may be included in commercially available materials, unless otherwise specified. All molecular weights as used herein are weight average molecular weights expressed as grams/moles, unless otherwise specified.

The term "amphiphile" means compositions which are both hydrophilic and lipophilic. Examples are provided below.

The term "gel network premix" as used herein means a fatty amphiphile and surfactant in a weight ratio ranging from 0.1:1 to 0.99:1 as discussed further below.

The terms "personal care components" as used herein means one or more components combined with a gel network premix to form the personal care composition. The components may be added individually or together to the gel network premix.

The term "personal care composition" or "final product" as used herein means the personal care components and gel network premixed combined together or the personal care components, gel network premix and any other composition combined together to form the complete material to be marketed as a personal care formula.

### Process

The subject of the present application is a process for preparing a personal care composition, where the process comprises combining a fatty amphiphile and surfactant in a fatty amphiphile to surfactant weight ratio from 0.1:1 to 0.99:1 to form a gel network premix at a temperature above the melt temperature of the fatty amphiphile. The process may also comprise contacting the gel network premix with a shearing device, and cooling the gel network premix after contact with the shearing device to a temperature below the melt temperature of the fatty amphiphile. Optionally, the process may include adding the cooled gel network premix to personal care components to form a personal care composition.

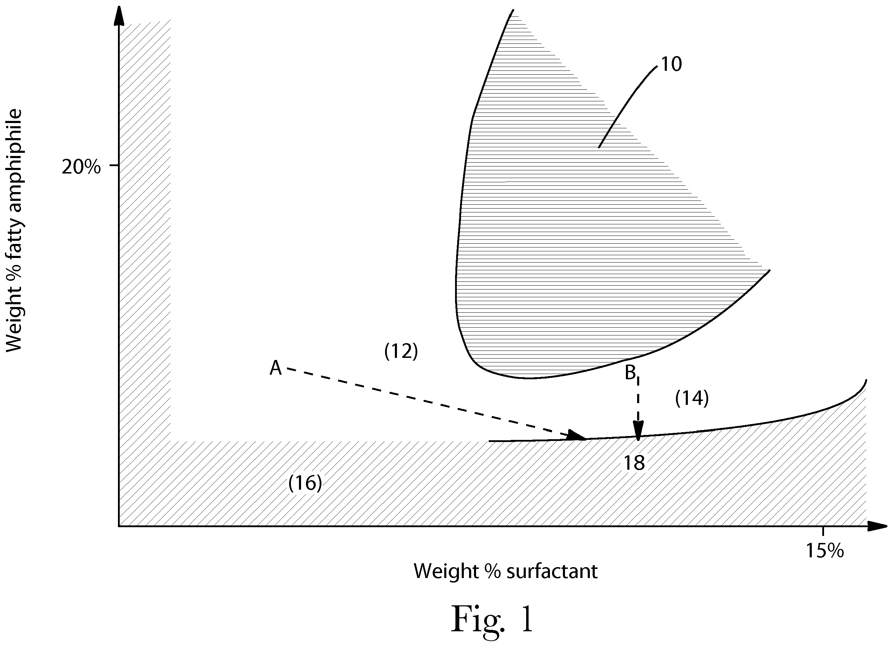

As generally illustrated and exemplified in the phase diagram of Fig. 1 (discussed in more detail below), a gel network premix forms a pure lamellar phase (10) if a threshold concentration of fatty amphiphile and surfactants are combined at an appropriate temperature. The width and length of the lamellar phase may depend on the particular amphiphile and surfactant utilized to form the gel network premix. For example, in a gel network premix with a higher relative concentration of fatty amphiphile (i.e. the compositions of the '744 patent) and represented by "A" in Fig. 1, there is equilibrium between the desired lamellar phase and a second phase (12), which has a much higher fatty amphiphile to surfactant ratio. Conversely, for a gel network premix having a higher relative concentration of surfactants (e.g., fatty amphiphile to surfactant ratio <1:1 and represented by "B" in Fig. 1), equilibrium exists between the lamellar phase and a surfactant-rich phase (14). The quantitative relationship amongst the various phases may be determined with standard analytical techniques, such as rheology, centrifugation, polarized microscopy, x-ray diffraction, and differential scanning calorimetry.

When producing gel network premixes with a fatty amphiphile to surfactant by weight ratios of less than unity, it is necessary to incorporate more surfactant into the liquid crystalline phase. Without being bound by theory, the shearing device helps disperse the viscous liquid crystalline phase comprising fatty amphiphile, water, and surfactant as it forms. This allows more surfactant to be incorporated into the fatty amphiphile during formation of the liquid crystalline phase. In low shear mixing processes or processes without mixing, the liquid crystalline phase may form before the desired amount of surfactant is incorporated therein. Without adequate incorporation of the surfactant into the liquid crystalline phase, the result would be a plurality of isolated gel structures or balls, instead of a desirable homogeneous viscous liquid.

A variety of ways of combining the fatty amphiphile and surfactant are contemplated. In one embodiment, the fatty amphiphile and surfactant are added simultaneously to an aqueous base to form a gel network premix. Alternatively, the fatty amphiphile and surfactant may be combined through a recirculation process, where either the fatty amphiphile or surfactant are added to the remaining component gradually to ensure adequate mixing, and the entire composition is circulated at least once through the shear device. The fatty amphiphile and surfactant may also be added individually, with one component being slowly added to the other component, during contact with a shear device. The gel network premix may also be contacted with the shear device in multiple passes. The gel network premix may be contacted with a shear device after the fatty amphiphile and surfactant are combined. However, other protocols for combining the fatty amphiphile and surfactants are also contemplated.

The shear device may comprise a static mixer, or a rotating dynamic mixer. Alternatively, the shear device may comprise a liquid whistle, or a high pressure homogeneous mixer, for example, the SONOLATOR™, commercially available from Sonic Corporation, Stratford, CT. In one configuration, the shear device is capable of imparting at least 100 J of energy per kg of material in 10 seconds or less. In one aspect, the shearing device is capable of imparting an amount of energy ranging from at least 5 J/Kg to 1000 J/kg. In another aspect, the shearing device is capable of imparting at least 5 J/kg to 1000 J/kg in a period of 10 seconds. It is also contemplated that other shearing energy inputs may be used in conjunction with the process disclosed herein.

The gel network premix components may be combined at a temperature sufficiently high to allow the surfactant and fatty amphiphile to form a lamellar gel phase. The fatty amphiphile and surfactant are combined at a temperature above the melt temperature of the fatty amphiphile or the melt temperature of a blend of fatty amphiphiles. The surfactant may be at a temperature from 5°C to 100°C lower than the temperature of the fatty amphiphile when combining the fatty amphiphile and surfactant to form the gel network premix. The fatty amphiphile(s) may have a melt temperature ranging from 30°C to 90°C, or the fatty amphiphile(s) may have a melt temperature ranging from 70°C to 90°C. It is understood that the melt temperature of the fatty amphiphile may vary depending on the particular fatty amphiphile or blend of fatty amphiphiles utilized in the composition. However, it is also contemplated that the gel network premix may also be processed at other temperatures not disclosed herein.

After contact with the shear device (i.e., after the components of the gel network premix are mixed), the gel network premix is cooled below the melt temperature of the fatty amphiphile(s). The gel network premix may be cooled using a variety of suitable methods and techniques as commonly understood in the art that do not interfere with the gel network formation. A heat exchanger may be used to cool the gel network premix. The heat exchanger may be of the shell-and-tube, or plate-and-frame type, with a non-contacting utility fluid. Alternatively, cooling could occur through contact with other ingredients in the composition provided at a lower temperature. In addition, the gel network premix may be cooled using a batch cooling process, utilizing a jacket or cooling coils. However, other cooling methods and devices are contemplated for the methods disclosed herein.

The cooled gel network premix has a viscosity ranging from 0.2 Pa.s (200 cp) to 5 Pa.s (5000 cp). The cooled gel network premix may have a viscosity ranging from 1Pa.s (1000 cp) to 4 Pa.s (4000 cp). However, it is also contemplated that the gel network premix may have other viscosities after it has been cooled. After the gel network premix has been sufficiently cooled, it may be added to personal care components to form a personal care composition. The viscosity may be determined using a shear rate of 1/sec at 26.7 °C, and a 75-mm cone/plate fixture on a Brookfield R/S viscometer. The cooled premix is added to personal care components less than 20 minutes after the gel network premix is cooled below the melt temperature. Alternatively, the cooled gel network premix may be added to personal care components at a time ranging from 20 minutes to 60 minutes after cooling. In addition, it is also contemplated that the gel network premix may be stored for durations from a few hours to several weeks. However, it is also contemplated that the gel network premix may be added at other durations after cooling to the personal care components than those disclosed herein.

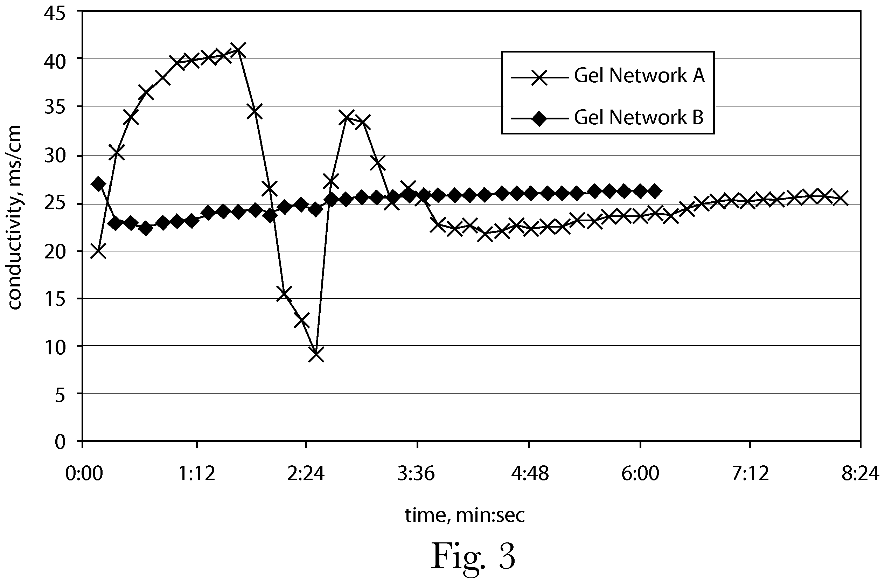

In operation, once the gel network premix is added to the personal care components, the gel network premix may equilibrate to the environment of the personal care composition featuring a much different FA:S ratio than in the gel network premix resulting from the personal care components combined with the gel network premix. Fig. 1 shows a generalized phase diagram for water, fatty amphiphile and surfactant at 80°C showing the various phases as a function of the gel network composition. The general behavior of this ternary systems is to form a highly viscous pure lamellar phase (10) for combinations that are sufficiently concentrated in the two non-aqueous components, and have a FA:S ratio within a certain range. The width and location of this FA:S range will depend on the particular fatty amphiphile and surfactant in the composition. It can be further seen from Fig. 3 the equilibration which must occur between the gel network premix (shown as "B" in Fig. 1) and the resulting personal care composition (18). A gel network premix featuring a fatty amphiphile to surfactant ratio below unity (i.e., more surfactant than fatty amphiphile is exemplified as "B" in Fig. 1) reduces the degree to which the gel network premix is out of equilibrium with the personal care components, therefore increasing the stability and robustness of the final personal care composition (18).

Comparatively, when the FA:S is greater than unity (i.e., more fatty amphiphile than surfactant and exemplified by "A" in Fig. 1), a gel network results in a mixture of fatty amphiphile droplets and lamellar phase (12), where not all of the fatty amphiphile is incorporated into the desired lamellar phase. Once cooled, an undesirably high rheology results for the gel network and the gel network can be a solid crystalline gel network increasing the difficulty in processing the gel network. The gel network exemplified by "A" in Fig. 1 shows the equilibrium path than is taken when the gel network is combined with personal care components (generally including at least anionic surfactants) to arrive at the personal care composition (18) having the ternary system resulting from the combination of the gel network and the personal care components.

### Examples

Referring to Table 2 below, Example 2 is a comparative example depicting the process and properties therewith of compositions produced from two gel premixes mixed with a personal care component. Gel Network A comprises a fatty amphiphile/surfactant (FA:S) ratio by weight greater than 1, specifically 2.54, and Gel Network B comprises an FA:S ratio by weight of less than 1, specifically 0.77, in accordance with the present composition.

**Table 2**

| | **Gel Network A** | **Gel Network B** |
|---|---|---|
| FA:S ratio | 2.54 | 0.77 |
| Stearyl alcohol (active wt.%) | 6.7 % | 6.7 % |
| Cetyl alcohol (active wt.%) | 3.7 % | 3.7 % |
| Sodium laureth-1 sulfate (active wt.%) | 4.1 % | 13.6 % |
| Viscosity (1/sec, 26.7°C) | 7.68 Pa.s (7680 cP) | 1.71 Pa.s (1710 cP) |
| Main DSC peak (heating, 3°C/min) | 64 C | 46 C |

As shown in Table 2, Gel Network B (FA:S ratio of 0.77) included 6.7 wt.% stearyl alcohol, and 3.7 wt.% cetyl alcohol as its fatty amphiphile, and 13.6 wt.% sodium laureth-1 sulfate as its surfactant. Using a shear rate of 1/sec at 26.7 °C, and a 75-mm cone/plate fixture on a Brookfield R/S viscometer, the viscosity of gel network B was 1.71 Pa.s (1710 cp). The main differential scanning calorimetry (DSC) peak with heating of 3°C/min was 46°C. These measurements were taken after a one week aging period at 25°C, since the gel network premix may take time to stabilize.

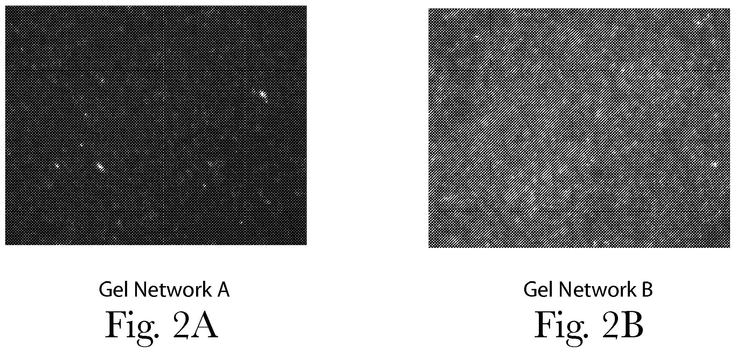

Figure 2A shows a microscope photograph of a cooled gel network resulting from the composition in Table 2 entitled "Gel Network A" having a FA:S ratio greater than unity (i.e., more fatty amphiphile than surfactant). The bright spots in Gel Network A clearly demonstrate the presence of fatty alcohol in addition to the desired gel network phase. This unincorporated fatty alcohol would not contribute efficiently to the desired conditioning benefit.

Figure 2B shows a microscope photograph of a cooled gel network resulting from the composition in Table 2 entitled "Gel Network B" having a FA:S ratio according to the present composition. Both microscope photographs were taken under cross-polar filters with a 40X objective on a Zeiss Axioskop 2 Plus microscope.

Referring to the graph of Fig. 3 comparing the kinetics of gel networks A and B when blended with the personal care components to form the personal care composition, Gel Network B demonstrates a faster and smoother equilibration. Mostly, the personal care components will be much richer in surfactant than in the fatty amphiphile. An exemplary (but in no means limiting) personal care components might comprise 11.5 wt.% active surfactant and 2 wt.% fatty amphiphile to achieve a desired balance of cleaning and conditioning. Therefore, upon addition of the gel network premix to the personal care components, the gel network premix must equilibrate to an environment with a much different FA:S ratio. For example, the above personal care components have an FA:S ratio of 0.17. As a result, the equilibration process can be slow and unpredictable, particularly when starting with a gel network premix that has an FA:S ratio above 1:1. Consequently, a gel network premix at FA:S ratios below 1:1 reduces the degree to which the gel network is out of equilibrium with its environment created when combined with personal care components, leading to a more robust and stable final personal care composition. The final product can be tracked using Differential Scanning Calorimetry (DSC) or another suitable technique.

Fig. 3 illustrates the enhanced kinetics of dispersing a gel network premix having a FA:S ratio ranging from 0.1:1 to 0.99:1 versus a gel network premix having a FA:S ratio of greater than 1:1. The conductivity probe (Mettler/Toledo InPro 7001 conductivity probe) identifies spikes in conductivity, which corresponds to gel-network-rich regions that have not yet blended with the personal care components in the personal care composition. After blending with a personal care components complementary to gel network A or B, respectively, the final formulation comprised 11.5 wt.% sodium laureth-1 sulfate, 1 wt.% cocoamidopropyl betaine, 1.5 wt.% ethylene glycol distearate, 1.8 wt.% stearyl alcohol, 1.0 wt.% cetyl alcohol, 0.5 wt.% sodium chloride, and 0.6 wt.% fragrance. In the current example, 161 g of gel network A or B was added to 439 g of the corresponding personal care components, and mixed in a 600mL vessel at 80 RPM and 26°C using a 2" pitched-blade turbine agitator. To monitor the dispersion kinetics, the conductivity of the mixture was measured at 10-second intervals using a conductivity probe inserted into the vessel. For both gel networks A and B, the DSC of the final composition peaks at 42°C, which is only 4°C away from the DSC peak of the Gel Network B, but a full 22°C below that of A.

Referring to Fig. 3 Gel Network B smoothly and quickly (within 2 minutes) approaches a well-mixed endpoint, and has a DSC peak at 46°C, very close to that of the final composition which peaks of 42°C (see Fig. 3). In contrast, Gel Network A takes a considerably long time (more than 6 minute) to disperse within the personal care components due to its high viscosity and surfactant-lean composition, as evidenced by its DSC peak at a much higher temperature of 64C.

Poor dispersibility for FA:S ratios greater than 1:1 is further illustrated by comparing the viscosities of the two exemplary gel network premixes. Since the gel network premix rheology can take a few hours to stabilize after making, the samples were allowed to age one week at 25 °C prior to the rheology measurement. As shown in Table 2, Gel Network Premix B has a lower viscosity, leading to easier handling at the manufacturing facility and easier dispersion into the final composition.

In addition to these benefits, the applicants also surprisingly discovered that gel network premixes having a FA:S ratio ranging from 0.1 to 0.99 yield a desirable low rheology. Increasing the surfactant level at a given fatty amphiphile level may increases the difficulty of making the gel network premix, as illustrated in Table 2 above; however, by improving the design of the process to make the hot premix, the applicants unexpectedly discovered that after cooling, these surfactant-rich premixes have a surprisingly low rheology, enabling easier pumping, handling, and dispersion of the premix. As used herein "easier" refers to materials having viscosities less than 5 Pa.s (5000 cP). The surfactant-rich premixes of the present disclosure are produced by contacting the fatty amphiphile and surfactant via a high-shear device such as a static mixer (e.g. SULZER SMX™, Sulzer Chemtech USA, Inc., Tulsa, OK) or rotating dynamic mixer (e.g. a BECOMIX™, A. Berents Gmbh & Co. KG, Germany). Although one might expect that a long contact time is needed to form the desired premix, the applicants have surprisingly found, in some embodiments, that a single pass through the high-shear device is sufficient to enable the invention.

### Personal Care Composition

As stated above, the personal care composition is produced by the blending of the gel network premix with the personal care components. Various compositional amounts for the gel network premix and the personal care components are contemplated. The final personal care composition comprisesfrom 10 wt% to 50 wt.% of the gel network premix.

As stated above, the gel network premix may be achieved by combining a fatty amphiphile and a surfactant in a FA:S weight ratio ranging from 0.75:1 to 0.9:1.

The ratio of FA: S in the gel network premix may be selected to be relatively close to the ratio of FA:S in the personal care composition. The FA:S in the personal care composition may be from 0.03:1 to 0.5:1.

### Fatty Amphiphile

Suitable fatty amphiphile components for use in the gel network premix herein include, but are not limited to, those which are known for use in hair care or other personal care compositions. Various compositional amounts are contemplated herein. The fatty amphiphile is included in the personal care composition at a level by weight ranging from 0.2 wt.% to 10 wt.%, or from 0.5 wt.% to 4 wt.%, or from 0.8 wt.% to 3 wt%.

A variety of different fatty amphiphiles are contemplated for use in preparing a gel network premix. The fatty amphiphile comprises 10 to 40 carbon atoms, or from 12 to 22 carbon atoms, or from 16 to 22 carbon atoms, or from 16 to 18 carbon atoms. The fatty amphiphile is straight or branched chain alcohols and is saturated or unsaturated.

The fatty amphiphile may include, but is not limited to, cetyl alcohol, stearyl alcohol, behenyl alcohol, and mixtures thereof. Mixtures of cetyl and stearyl alcohol in a ratio of from 20:80 to 80:20 are also contemplated. The fatty amphiphile may comprise up to 50 wt% by weight of the gel network premix of stearyl alcohol. However, other fatty amphiphiles and combinations thereof are also contemplated for use in the present disclosure.

### Surfactants

In specific embodiments, it is contemplated that the weight percentage is calculated in terms of the active component of the surfactant ingredient. By "active," it means the portion of the surfactant component that is not water, fillers, salts, buffers, etc. For example, a 25 wt.% active sodium lauryl sulfate may also contain 74 wt.% of water, and 1 wt.% of unreacted lauryl alcohol, salts, pH buffers, etc. The surfactant component may be selected from the group comprising anionic, zwitterionic or amphoteric surfactants, or a combination thereof. Alternatively, the surfactant component may comprise other types of surfactants suitable for use in the gel network premix and personal care components.

It is also contemplated that, in some embodiments, certain types of surfactants may not be added to the gel network premix composition directly, but rather to the personal care components, to avoid interference with the formation of the gel network in the gel network premix composition. Each surfactant may be studied on an individual basis to assess whether it can be added directly to the gel network premix without compromising the gel network structure.

Suitable surfactant components for use in the composition herein include those which are known for use in hair care or other personal care cleansing compositions. The concentration of the surfactant component may be sufficient to provide the desired cleaning and lather performance. The active surfactant component may be in the range from 5 wt.% to 50 wt.%, or 8 wt.% to 30 wt.%, or from 12 wt.% to 22 wt.% of the personal care composition.

It is contemplated that a variety of surfactants may be used in forming the gel network premix. The surfactants suitable for use in the compositions are the alkyl and alkyl ether sulfates. These materials have the respective formulae ROSO₃₋ and RO(C₂H₄O)ₓSO₃₋M, wherein R is alkyl or alkenyl of from 8 carbon atoms to 18 carbon atoms, x is an integer having a value of from 1 to 10, and M is a cation such as ammonium, alkanolamines, such as triethanolamine, monovalent metals, such as sodium and potassium, and polyvalent metal cations, such as magnesium, and calcium. R may include from 8 carbon atoms to 18 carbon atoms, or from 10 carbon atoms to 16 carbon atoms, or from 12 carbon atoms to 14 carbon atoms, in both the alkyl and alkyl ether sulfates. The alkyl ether sulfates are typically made as condensation products of ethylene oxide and monohydric alcohols having from 8 carbon atoms to 24 carbon atoms. The alcohols can be synthetic or they can be derived from fats, e.g., coconut oil, palm kernel oil, and tallow. Lauryl alcohol and straight chain alcohols derived from coconut oil or palm kernel oil may be utilized. Such alcohols are reacted with between 0 and 10, or from 2 to 5, or from 3, molar proportions of ethylene oxide, and the resulting mixture of molecular species having, for example, ethoxylate levels ranging from 0 moieties per molecule to 4 moieties per molecule. IAn average of 1 mole of ethylene oxide per mole of alcohol may be sulfated and neutralized.

The surfactant is selected from the group consisting of alkyl sulfate, alkyl ether sulfate, and combinations thereof. Nonlimiting examples off anionic surfactants for use in the compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof. It is also contemplated that suitable amphoteric or zwitterionic surfactants may be utilized in performing the process described herein, including those which are known for use in hair care or other personal care cleansing. Concentration of such amphoteric surfactants may include ranges from 0.5 wt.% to 20 wt.%, or from 1 wt.% to 10 wt.% of the personal care composition. Non limiting examples of suitable zwitterionic or amphoteric surfactants are described in U.S. 5,104,646 (Bolich Jr. et al.), U.S. 5,106,609 (Bolich Jr. et al.).

Alternatively, the surfactant may comprise amphoteric surfactants, which are broadly defined as derivatives of aliphatic secondary and tertiary amines in which the aliphatic radical can be straight or branched chain and wherein one of the aliphatic substituents contains from 8 carbon atoms to 18 carbon atoms and one contains an anionic group such as carboxy, sulfonate, sulfate, phosphate, or phosphonate. The amphoteric surfactants may include cocoamphoacetate, cocoamphodiacetate, lauroamphoacetate, lauroamphodiacetate, and mixtures thereof.

Zwitterionic surfactants may also be suitable for use in the personal care composition and are well known in the art, and include those surfactants broadly described as derivatives of aliphatic quaternary ammonium, phosphonium, and sulfonium compounds, in which the aliphatic radicals can be straight or branched chain, and wherein one of the aliphatic substituents contains from 8 carbon atoms to 18 carbon atoms and one contains an anionic group such as carboxy, sulfonate, sulfate, phosphate or phosphonate. The zwitterionic surfactant may comprise betaine.

The compositions of the present disclosure may further comprise additional surfactants for use in combination with the anionic surfactant component described herein. Suitable optional surfactants include nonionic and cationic surfactants. The concentration of the optional additional surfactants in the composition may vary with the cleansing or lather performance desired, the optional surfactant selected, the desired product concentration, the presence of other components in the composition, and other factors well known in the art.

Nonionic surfactants which are useful in the present disclosure include those broadly defined as compounds produced by the condensation of alkylene oxide groups (hydrophilic in nature) with an organic hydrophobic compound, which may be aliphatic or alkyl aromatic in nature.
Exemplary cationic surfactants may be those containing at least one alkyl chain having at least 16 carbon atoms. Nonlimiting examples of cationic surfactants include: behenyl trimethyl ammonium chloride available, for example, with tradename INCROQUAT TMC-80 from Croda and ECONOL TM22 from Sanyo Kasei; cetyl trimethyl ammonium chloride available, for example, with tradename CA-2350 from Nikko Chemicals, hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, di(behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyl colamino formyl methyl) pyridinium chloride.

Optional components useful as personal care components for use in the personal care composition may include, but are not limited to, cationic polymers, conditioning agents (hydrocarbon oils, fatty esters, silicones), anti dandruff agents, suspending agents, viscosity modifiers, dyes, nonvolatile solvents or diluents (water soluble and insoluble), pearlescent aids, foam boosters, additional surfactants or nonionic co-surfactants, pediculocides, pH adjusting agents, perfumes, preservatives, chelants, proteins, skin active agents, sunscreens, UV absorbers, and vitamins.

The personal care compositions of the present disclosure may include a deposition aid. The deposition aid is included to effectively enhance deposition of the fatty amphiphile gel network premix described previously. The deposition aid can comprise any material that enhances the deposition of the fatty amphiphile gel network from the shampoo onto the hair and/or scalp. For example, the deposition aids are cationic polymers. The concentration of the deposition aid in the personal care composition may be sufficient to effectively enhance the deposition of the fatty amphiphile gel network component and may range from 0.05 wt.% to 5 wt.%, or from 0.075 wt.% to 2.5 wt.%, or from 0.1 wt.% to 1.0 wt.%, of the personal care composition.

The compositions of the present disclosure may contain a cationic polymer. Concentrations of the cationic polymer in the composition typically range from 0.05 wt.% to 3 wt.%, or from 0.075 wt.% to 2.0 wt.%, or from 0.1 wt.% to 1.0 wt.%, of the personal care composition. Non-limiting examples of cationic polymers will have cationic charge densities of at least 0.9 meq/gm, or at least 1.2 meq/gm, or at least 1.5 meq/gm, or less than 7 meq/gm, or less than 5 meq/gm, at the pH of intended use of the composition, which pH will generally range from a pH of 3 to 9, or between a pH of 4 to 8. The "cationic charge density" of a polymer, as that term is used herein, refers to the ratio of the number of positive charges on the polymer to the molecular weight of the polymer. The average molecular weight of such suitable cationic polymers may range between 10,000 and 10 million, or between 50,000 and 5 million, or between 100,000 and 3 million.

Suitable cationic polymers for use in the compositions of the present disclosure contain cationic nitrogen-containing moieties such as quaternary ammonium or cationic protonated amino moieties. The cationic protonated amines can be primary, secondary, or tertiary amines (in, specific embodiments, secondary or tertiary), depending upon the particular species and the selected pH of the composition. Any anionic counterions can be used in association with the cationic polymers so long as the polymers remain soluble in water, in the composition, or in a coacervate phase of the personal care composition. Non limiting examples of such counterions include halides (e.g., chloride, fluoride, bromide, and/or iodide), sulfate and methylsulfate. However, other suitable cationic polymers are also contemplated for use in the present disclosure. Polyalkylene glycols having a molecular weight of more than 1000 are useful herein. Useful are those having the following general formula: 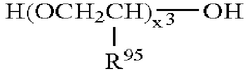 wherein R ⁹⁵ is selected from the group consisting of hydrogen, methyl, and mixtures thereof. Polyethylene glycol polymers useful herein are PEG-2M (also known as POLYOX WSR® N-10, which is available from Union Carbide and as PEG-2,000); PEG-5M (also known as POLYOX WSR® N-35 and POLYOX WSR® N-80, available from Union Carbide and as PEG-5,000 and Polyethylene Glycol 300,000); PEG-7M (also known as POLYOX WSR® N-750 available from Union Carbide); PEG-9M (also known as POLYOX WSR® N-3333 available from Union Carbide); and PEG-14 M (also known as POLYOX WSR® N-3000 available from Union Carbide). Conditioning agents include any material which is used to give a particular conditioning benefit to hair and/or skin. In hair treatment compositions, suitable conditioning agents are those which deliver one or more benefits relating to shine, softness, comb-ability, antistatic properties, wet-handling, damage, manageability, body, and greasiness. The conditioning agents useful in the personal care compositions typically comprise water insoluble, water dispersible, non-volatile, liquid that forms emulsified, liquid particles or are solubilized by the surfactant micelles, in a anionic surfactant.
Suitable conditioning agents for use in the personal care composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein.

The concentration of the conditioning agent in the personal care composition may be sufficient to provide the desired conditioning benefits, and as will be apparent to one of ordinary skill in the art. Such concentration can vary with the conditioning agent, the conditioning performance desired, the average size of the conditioning agent particles, the type and concentration of other components, and other like factors.

The conditioning agent of the personal care compositions may also include an insoluble silicone conditioning agent. The silicone conditioning agent particles may comprise volatile silicone, non-volatile silicone, or combinations thereof. The conditioning agent may be a non-volatile silicone conditioning agent. If volatile silicones are present, it will typically be incidental to their use as a solvent or carrier for commercially available forms of non-volatile silicone materials ingredients, such as silicone gums and resins. The silicone conditioning agent particles may comprise a silicone fluid conditioning agent and may also comprise other ingredients, such as a silicone resin to improve silicone fluid deposition efficiency or enhance glossiness of the hair.

Suitable organic conditioning oils for use as conditioning agents in the personal care compositions include, but are not limited to, hydrocarbon oils having at least 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils may be from C₁₂ to C₁₉. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2,2,4,4,6,6,8,8-dimethyl-10-methylundecane and 2,2,4,4,6,6-dimethyl-8-methylnonane, available from Permethyl Corporation. Hydrocarbon polymers such as polybutene and polydecene may be used. The hydrocarbon polymer may be polybutene, i.e., the copolymer of isobutylene and butene. A commercially available material of this type is L-14 polybutene from Amoco Chemical Corporation. The concentration of such hydrocarbon oils in the composition may range from 0.05 wt.% to 20 wt.%, or from 0.08 wt.% to 1.5 wt.%, or from 0.1 wt.% to 1 wt.% of the composition.

The personal care composition of the present disclosure may include dispersed particles. The present compositions may include 0.025 wt.% of the dispersed particles, or at least 0.05 wt.%, or at least 0.1 wt.%, or at least 0.25 wt.%, or at least 0.5 wt.% of the dispersed particles. The personal care compositions may include no more than 20 wt.% of the dispersed particles, or no more than 10 wt.%, or no more than 5 wt.%, or no more than 3 wt.%, or no more than 2 wt.% of the dispersed particles.

Suitable dispersed particles include anti-dandruff agents. Suitable, non-limiting examples of anti-dandruff particulates include: pyridinethione salts, azoles, selenium sulfide, particulate sulfur, keratolytic agents, and mixtures thereof. Such anti-dandruff actives should be physically and chemically compatible with the essential components of the composition, and should not otherwise unduly impair product stability, aesthetics or performance. When present in the personal care composition, the anti-dandruff active may be included in an amount from 0.01% to 5%, from 0.1% to 3%, and from 0.3% to 2%, by weight of the composition.

In addition to the anti-dandruff active selected from polyvalent metal salts of pyrithione, the present disclosure may further comprise one or more anti-fungal or anti-microbial actives in addition to the metal pyrithione salt actives. Suitable anti-microbial actives include coal tar, sulfur, Whitfield's Ointment, Castellani's Paint, aluminum chloride, gentian violet, octopirox (piroctone olamine), ciclopirox olamine, undecylenic acid and it's metal salts, potassium permanganate, selenium sulfide, sodium thiosulfate, propylene glycol, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone and azoles, and combinations thereof. The anti-microbials may comprise itraconazole, ketoconazole, selenium sulfide, coal tar, or combinations thereof.

The personal care compositions may also contain a humectant. The humectants herein are selected from the group consisting of polyhydric alcohols, water soluble alkoxylated nonionic polymers, and mixtures thereof. The humectants, when used herein, may be present at levels from 0.1 wt.% to 20 wt.%, or from 0.5 wt.% to 5 wt.%.
The personal care compositions may further comprise a suspending agent at concentrations effective for suspending water-insoluble material in dispersed form in the compositions or for modifying the viscosity of the composition. Such concentrations may range from 0.1 wt.% to 10 wt.%, or from 0.3 wt.% to 5.0 wt.%, of the compositions. Suspending agents useful herein include, but are not limited to, anionic polymers and nonionic polymers. Useful herein may be vinyl polymers such as cross linked acrylic acid polymers such as carbomers, cellulose derivatives and modified cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carragheenin, pectin, agar, quince seed (*Cydonia oblonga* Mill), starch (rice, corn, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

The personal care compositions may contain also vitamins and amino acids such as: water soluble vitamins such as vitamin B1, B2, B6, B12, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

The personal care compositions may also contain pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, and natural colors.

The personal care compositions may also contain antimicrobial agents, which are useful as cosmetic biocides and antidandruff agents including: water soluble components such as piroctone olamine, water insoluble components such as 3,4,4-trichlorocarbanilide (trichlosan), triclocarban and zinc pyrithione. The personal care compositions may also contain chelating agents.

It is noted that terms like "preferably," "commonly," and "typically," when utilized herein, are not utilized to limit the scope of the claimed disclosure or to imply that certain features are critical, essential, or even important to the structure or function of the claimed disclosure. Rather, these terms are merely intended to identify particular aspects of an embodiment of the present disclosure or to emphasize alternative or additional features that may or may not be utilized in a particular embodiment of the present disclosure.

For the purposes of describing and defining the present disclosure it is noted that the terms "substantially" and "approximately" are utilized herein to represent the inherent degree of uncertainty that may be attributed to any quantitative comparison, value, measurement, or other representation. The terms "substantially" and "approximately" are also utilized herein to represent the degree by which a quantitative representation may vary from a stated reference without resulting in a change in the basic function of the subject matter at issue.

## Claims

1. A process for preparing a personal care composition, said process comprising:
combining a fatty amphiphile above its melt temperature with a surfactant in a fatty amphiphile to surfactant weight ratio from 0.75:1 to 0.9:1 to form a gel network premix,
wherein the fatty amphiphile and surfactant are combined at a temperature above the melt temperature of the fatty amphiphile or the melt temperature of a blend of fatty amphiphiles,
wherein the fatty amphiphile comprises from 10 to 40 carbon atoms, and wherein the fatty amphiphile is straight or branched chain alcohols and are saturated or unsaturated,
wherein the surfactant is selected from the group consisting of alkyl sulfate, alkyl ether sulfate, and combinations thereof;
contacting the gel network premix with a shearing device;
cooling the gel network premix after contact with the shearing device to a temperature below the melt temperature of the fatty amphiphile; and
adding the cooled gel network premix to one or more personal care components to form the personal care composition, wherein the final personal care composition comprises from 10 wt% to 50 wt% of the gel network premix.

2. The process of claim 1, wherein the shearing device is capable of imparting an amount of energy ranging from at least 5 J/Kg to 1000 J/Kg.

3. The process of claim 1, wherein the contacting the gel network premix with a shearing device step has a duration of less than 10 seconds.

4. The process of claim 1, wherein the combining step results in the formation of the gel network premix in a lamellar gel phase.

5. The process of claim 1, wherein the surfactant is at a temperature from 5°C to 100°C lower than the temperature of the fatty amphiphile when combining the fatty amphiphile and surfactant to form the gel network premix, preferably wherein the temperature of the fatty amphiphile has a melt temperature ranging from 70°C to 90°C.

6. The process of claim 1, wherein the fatty amphiphile is selected from the group consisting of stearyl alcohol, cetyl alcohol, behenyl alcohol, and mixtures thereof.

7. The process of claim 1, wherein the gel network premix comprises from 5 wt.% to 50 wt.% by weight of gel network fatty amphiphile.

8. The process of claim 1, wherein the gel network premix comprises from 5 wt.% to 50 wt.% by weight of gel network active surfactant.

9. The process of claim 1, wherein the cooled gel network premix has a viscosity ranging from 0.2 Pa.s (200 cP) to 5 Pa.s (5000 cP) at 26.7°C and a shear rate of 1/sec.

10. The process of claim 1, wherein the cooled gel network premix is added to one or more personal care components less than 20 minutes after cooling the gel network premix below the melt temperature of the fatty amphiphile.

## Patentansprüche

1. Verfahren zur Herstellung einer Körperpflegezusammensetzung, wobei das Verfahren umfasst:
Kombinieren eines Fettamphiphils oberhalb seiner Schmelztemperatur mit einem Tensid in einem Fettamphiphil-zu-Tensid-Gewichtsverhältnis von 0,75:1 bis 0,9:1, um eine Gelnetzvormischung zu bilden,
wobei das Fettamphiphil und das Tensid bei einer Temperatur von oberhalb der Schmelztemperatur des Fettamphiphils oder der Schmelztemperatur einer Mischung von Fettamphiphilen miteinander kombiniert werden,
wobei das Fettamphiphil zu 10 bis 40 Kohlenstoffatome umfasst und wobei das Fettamphiphil aus gerad- oder verzweigtkettigen Alkoholen besteht und gesättigt oder ungesättigt ist,
wobei das Tensid ausgewählt ist aus der Gruppe, bestehend aus Alkylsulfat, Alkylethersulfat und Kombinationen davon;
Inkontaktbringen der Gelnetzvormischung mit einer Schervorrichtung;
Abkühlen der Gelnetzvormischung nach Inkontaktbringen mit der Schervorrichtung auf eine Temperatur von unterhalb der Schmelztemperatur des Fettamphiphils; und
Hinzufügen der gekühlten Gelnetzvormischung zu einer oder mehreren Körperpflegekomponenten, um die Körperpflegezusammensetzung zu bilden, wobei die endgültige Körperpflegezusammensetzung zu 10 Gew.- % bis 50 Gew.- % die Gelnetzvormischung umfasst.

2. Verfahren nach Anspruch 1, wobei die Schervorrichtung in der Lage ist, eine Energiemenge im Bereich von mindestens 5 J/kg bis 1000 J/kg zu vermitteln.

3. Verfahren nach Anspruch 1, wobei das Inkontaktbringen der Gelnetzvormischung mit einem Schervorrichtungsschritt eine Dauer von weniger als 10 Sekunden aufweist.

4. Verfahren von Anspruch 1, wobei der Schritt des Kombinierens zur Bildung der Gelnetzvormischung in einer lamellaren Gelphase führt.

5. Verfahren nach Anspruch 1, wobei das Tensid eine Temperatur von 5 °C bis 100 °C niedriger als die Temperatur des Fettamphiphils aufweist, wenn das Fettamphiphil und das Tensid miteinander kombiniert werden, um die Gelnetzvormischung zu bilden, wobei die Temperatur des Fettamphiphils vorzugsweise eine Schmelztemperatur im Bereich von 70 °C bis 90 °C aufweist.

6. Reinigungszusammensetzung nach Anspruch 1, wobei das Fettamphiphil ausgewählt ist aus der Gruppe, bestehend aus Stearylalkohol, Cetylalkohol, Behenylalkohol und Mischungen davon.

7. Verfahren nach Anspruch 1, wobei die Gelnetzvormischung zu 5 Gew.-% bis 50 Gew.-% nach Gewicht das Gelnetzfettamphiphil umfasst.

8. Verfahren nach Anspruch 1, wobei die Gelnetzvormischung zu 5 Gew.-% bis 50 Gew.-% nach Gewicht aktives Gelnetztensid umfasst.

9. Verfahren nach Anspruch 1, wobei die gekühlte Gelnetzvormischung eine Viskosität im Bereich von 0,2 Pas (200 cP) bis 5 Pas (5000 cP) bei 26,7 °C und eine Scherrate von 1/s aufweist.

10. Verfahren nach Anspruch 1, wobei die gekühlte Gelnetzvormischung weniger als 20 Minuten nach dem Abkühlen der Gelnetzvormischung auf unterhalb der Schmelztemperatur des Fettamphiphils zu einer oder mehreren Körperpflegekomponenten zugegeben wird.

## Revendications

1. Procédé de préparation d'une composition de soins personnels, ledit procédé comprenant :
la combinaison d'un amphiphile gras au-dessus de sa température de fusion avec un agent tensioactif selon un rapport pondéral de l'amphiphile gras à l'agent tensioactif allant de 0,75:1 à 0,9:1 pour former un prémélange de réseau de gel,
dans lequel l'amphiphile gras et l'agent tensioactif sont combinés à une température au-dessus de la température de fusion de l'amphiphile gras ou de la température de fusion d'un mélange d'amphiphiles gras,
dans lequel l'amphiphile gras comprend de 10 à 40 atomes de carbone, et dans lequel l'amphiphile gras est des alcools à chaîne linéaire ou ramifiée et sont saturés ou insaturés,
dans lequel l'agent tensioactif est choisi dans le groupe constitué d'alkyl sulfate, d'alkyl éther sulfate, et de leurs combinaisons ;
la mise en contact du prémélange de réseau de gel avec un dispositif de cisaillement ;
le refroidissement du prémélange de réseau de gel après contact avec le dispositif de cisaillement à une température en dessous de la température de fusion de l'amphiphile gras ; et
l'ajout du prémélange de réseau de gel refroidi à un ou plusieurs composants de soins personnels pour former la composition de soins personnels, dans lequel la composition de soins personnels finale comprend de 10 % en poids à 50 % en poids du prémélange de réseau de gel.

2. Procédé selon la revendication 1, dans lequel le dispositif de cisaillement est capable de conférer une quantité d'énergie se situant dans une plage allant d'au moins 5 J/kg à 1 000 J/kg.

3. Procédé selon la revendication 1, dans lequel l'étape de mise en contact du prémélange de réseau de gel avec un dispositif de cisaillement a une durée inférieure à 10 secondes.

4. Procédé selon la revendication 1, dans lequel l'étape de combinaison conduit à la formation du prémélange de réseau de gel dans une phase de gel lamellaire.

5. Procédé selon la revendication 1, dans lequel l'agent tensioactif se trouve à une température de 5 °C à 100 °C inférieure à la température de l'amphiphile gras lors de la combinaison de l'amphiphile gras et de l'agent tensioactif pour former le prémélange de réseau de gel, de préférence dans lequel la température de l'amphiphile gras a une température de fusion se situant dans une plage allant de 70 °C à 90 °C.

6. Procédé selon la revendication 1, dans lequel l'amphiphile gras est choisi dans le groupe constitué de l'alcool stéarylique, de l'alcool cétylique, de l'alcool béhénylique, et de leurs mélanges.

7. Procédé selon la revendication 1, dans lequel le prémélange de réseau de gel comprend de 5 % en poids à 50 % en poids d'amphiphile gras de réseau de gel.

8. Procédé selon la revendication 1, dans lequel le prémélange de réseau de gel comprend de 5 % en poids à 50 % en poids d'agent tensioactif de réseau de gel.

9. Procédé selon la revendication 1, dans lequel le prémélange de réseau de gel refroidi a une viscosité se situant dans une plage allant de 0,2 Pa.s (200 cP) à 5 Pa.s (5 000 cP) à 26,7 °C et un taux de cisaillement de 1/sec.

10. Procédé selon la revendication 1, dans lequel le prémélange de réseau de gel refroidi est ajouté à un ou plusieurs composants de soins personnels moins de 20 minutes après le refroidissement du prémélange de réseau de gel en dessous de la température de fusion de l'amphiphile gras.
